(19) Europäisches Patentamt
European Patent Office
Office européen des brevets

(11) EP 0 623 580 B1

(12) **EUROPÄISCHE PATENTSCHRIFT**

(45) Veröffentlichungstag und Bekanntmachung des Hinweises auf die Patenterteilung:
**26.03.1997 Patentblatt 1997/13**

(51) Int Cl.[6]: **C07C 59/215**, C12P 7/42, C07D 307/60

(21) Anmeldenummer: **94106577.3**

(22) Anmeldetag: **27.04.1994**

(54) **2-Keto-3-methyl-4-hydroxy-valeriansäure**

2-ceto-3-methyl-4-hydroxy-valeric acid

Acide ceto-2-methyl-3-hydroxy-4-valérique

(84) Benannte Vertragsstaaten:
**BE CH DE DK ES FR GB IT LI NL SE**

(30) Priorität: **06.05.1993 CH 1394/93**

(43) Veröffentlichungstag der Anmeldung:
**09.11.1994 Patentblatt 1994/45**

(73) Patentinhaber: **GIVAUDAN-ROURE (INTERNATIONAL) S.A.**
**CH-1214 Vernier, Genève (CH)**

(72) Erfinder: **Lerch, Konrad**
**CH-8006 Zürich (CH)**

(74) Vertreter: **Urech, Peter, Dr. et al**
**F.Hoffmann-La Roche AG**
**Patent Department (PLP),**
**124 Grenzacherstrasse**
**4070 Basel (CH)**

(56) Entgegenhaltungen:
**FR-A- 2 022 013** **US-A- 4 357 425**

- **JOURNAL OF ORGANIC CHEMISTRY., Bd. 49, Nr. 15, 27.Juli 1984, EASTON US, Seiten 2714-2718, ROBERT F. RAFFAUF ET AL.: "Funebrine, a structurally novel pyrrole alkaloid,..."**
- **PHYTOCHEMISTRY, Bd. 12, Nr. 7, 1973, PERGAMON PRESS, GB, Seiten 1707-1711, LESLIE FOWDEN ET AL.: "4-Hydroxyisoleucine from seed of trigonella foenum-graecum"**
- **ZEITSCHRIFT FÜR LEBENSMITTEL-UNTERSUCHUNG UND -FORSCHUNG, Bd. 148, Nr. 4, Mai 1972, SPRINGER-VERLAG, BERLIN . HEIDELBERG . NEW YORK, Seiten 215-221, H. SULSER ET AL.: "Synthese und Geschmacksprüfungen von 3,4-disubstituierten 2-Hydroxy-2-buten-1,4-oliden"**
- **TETRAHEDRAON LETTERS, 1992, Bd 33, No 38, S.5625-28**
- **CHEMICAL ABSTRACTS, Bd 99, 1983, Abstract No 122204j**
- **CHEMICAL ABSTRACTS, Bd 99, 1983, Abstract No 153825s**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 623 580 B1

Printed by Jouve, 75001 PARIS (FR)

## Beschreibung

Die Erfindung betrifft zwei neue, optisch aktive Formen der 2-Keto-3-methyl-4-hydroxy-valeriansäure der Formel

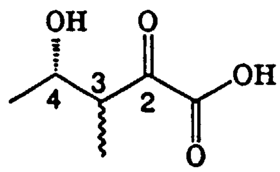

(I)

wobei das C-Atom 4 die (S)-Konfiguration aufweist. Das C-Atom 3 kann sowohl in der (R)- als auch der (S)-Konfiguration vorliegen.

Die Erfindung betrifft auch ein mikrobiologisches Verfahren zur Herstellung der Verbindungen (I).

Dieses Verfahren ist dadurch gekennzeichnet, dass man 4-Hydroxy-isoleucin (2S,3R,4S) (= L-4-Hydroxyisoleucin) der Formel

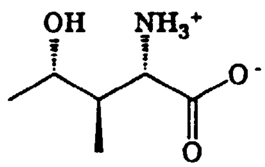

(II)

mit Mikroorganismen, die L-Aminosäureoxidase (EC 1.4.3.2.)-Aktivität besitzen, umsetzt oder mit einer L-Aminosäureoxidase umsetzt. *

Die Verbindungen(I)sind interessante Zwischenprodukte zur Herstellung des optisch aktiven, des (5S)-3-Hydroxy-4,5-dimethyl-2(5H)-furanons der Formel

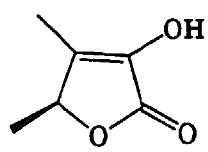

(III)

Das Racemat von (III) ist eine wichtige Aromasubstanz mit einem extrem tiefen Schwellenwert (H. Sulser, M. Habegger, W. Büchi, *Z. Lebensm. Unters. Forsch.* (1972), **148**, 215).

Das vorliegende Verfahren erlaubt also, (III) aus einem natürlichen Rohmaterial mit Hilfe eines als natürlich geltenden Prozesses herzustellen.

Die in Tetrahedron Letters 33 (38), 5625 seq. (1992) beschriebene Synthese andererseits ist keine solche Synthese, weil sie nicht von einem Naturprodukt als Ausgangsmaterial Gebrauch macht, und des weitern neben einer enzymatischen Stufe auch mehrere chemische Stufen umfasst, und schliesslich nur zu einem Produkt führt, das "enantiomerically enriched" ist.

Auch für die in C.A. 99 (15) 122204j (1983), (Agric. Biol. Chem. 47 (5), 1071 seq. (1983)) beschriebene Synthese gilt wegen des Einsatzes der klassichen Chemie das Kriterium des als natürlich geltenden Prozesses nicht.

C.A. 99 (19) 153825s (1983) schliesslich berichtet nur über die Wirksamkeit, unter anderem des fraglichen Isomeren, als Insektenlockstoff.

Es gelang also, ein Verfahren zu entwickeln, das die Gewinnung dieser Substanz III auf biotechnologischem Weg erlaubt.

Als Rohmaterial für (I) bzw. (III) eignen sich insbesondere Samen von Bockshornklee *Trigonellum foenum graecum L.*

Da die Samen von Bockshornklee relativ grosse Mengen (5 g / kg Samen) freies L-4-Hydroxyisoleucin enthalten (L. Fowden, H.M. Pratt, A. Smith, *Phytochemistry* (1973), **12**, 1707), kann die in den Samen von Bockshornklee vorhandene freie Aminosäure (II) mit Hilfe des aufgefundenen Biotransformationssystems auf einfache Weise zunächst

* USP 4 357425 beschreibt eine L-Aminosäureoxidase und deren Herstellung.

in (I) übergeführt werden.

Dies gelingt dadurch, dass man, wie vorne ausgeführt, (II) mit Mikroorganismen, die L-Aminosäureoxidase (EC 1.4.3.2.) Aktivität besitzen, umsetzt oder L-4-Hydroxyisoleucin mit einer L-Aminosäureoxidase selbst umsetzt.

Die Umsetzung verläuft also wie folgt:

$$II + O_2 + H_2O \rightarrow I + NH_3 + H_2O_2$$

Die dabei entstehenden diastereomeren Formen von 2-Keto-3-methyl-4-hydroxy-valeriansäure (I) können z.B auf einfache Weise als Metallsalze, wie die Alkalimetall- oder Erdalkalimetallsalze, wie das Natrium- oder Kaliumsalz, isoliert werden. Die freien Säuren sind nämlich sehr labil und zyklisieren spontan zum optisch aktiven Lakton (III).

Für die Biotransformation von (II) eignen sich die verschiedensten Mikroorganismen, wie Bakterien und Pilze, und Algen, welche L-4-Hydroxrisoleucin in (I) umwandeln können.

Bevorzugte Organismen sind solche mit hoher Aktivität an L-Aminosäureoxidase. Dazu gehören Algen der Gattung *Amphiroa*, *Gymnogongrus* u.a.; Pilze der Gattung *Neurospora* u.a.; Bakterien der Gattung *Proteus*, *Providencia*, *Morganella*, *Corynebacterium* u.a. Besonders gut geeignet sind Bakterien der Gattung *Proteus*, *Providencia* und *Morganella*, z.B.

| | |
|---|---|
| *Proteus vulgaris* | (DSM 2140,3265) |
| *Proteus mirabilis* | (ATCC 15290) |
| *Providencia rettgeri* | (ATCC 9250) |
| *Morganella morganii* | (DSM 30117) |

oder Pilze der Gattung *Neurospora* wie z. B.

*Neurospora crassa* (FGSC 988 und 2963); alle diese Mikro-organismen sind der Oeffentlichkeit zugänglich

Die Mikroorganismen werden vor der eigentlichen Biotransformation zweckmässigerweise in Submerskultur auf einem geeigneten Nährmedium gezüchtet. Dazu können sowohl die üblichen komplexen als auch chemisch definierten Medien verwendet werden, vorausgesetzt, es findet eine Neusynthese der für die Biotransformation nötigen L-Aminosäureoxidase statt. Die entsprechende Technik ist dem Fachmann auf dem betreffenden Gebiet bekannt. Die Nährmedien enthalten neben geeigneten Kohlenstoff- und Stickstoffquellen zweckmässigerweise anorganische Salze, Spurenelemente und freie Aminosäuren.

Als Kohlenstoffquellen verwendet man beispielsweise Zucker, vorzugsweise Glucose oder Saccharose. Als Stickstoffquelle kommen sowohl anorganische wie organische Verbindungen in Betracht, beispielsweise Ammoniumsalze, vorzugsweise Ammoniumsulfat und Ammoniumnitrat, Nitrate, Hefeextrakt oder Peptone usw.

Ueblicherweise enthält das Nährmedium noch Salze, z.B. Sulfate, Phosphate oder Chloride, insbesondere der Elemente Magnesium, Kalium und Calcium, sowie die Spurenelemente Eisen, Zink, Mangan, Bor, Kobalt, Kupfer und Molybdän. Im Fall von *Neurospora crassa* ist die Zugabe des Vitamins Biotin unentbehrlich.

Die verwendeten freien Aminosäuren stammen zweckmässigerweise aus Proteinhydrolysaten, vorzugsweise aus Casein- und Soyaproteinhydrolysaten oder bei einzelnen Aminosäuren aus entsprechenden Fermentationen. Bevorzugt sind dabei Leucin, Methionin und Phenylalanin.

Das Mengenverhältnis der genannten Nährstoffe hängt zweckmässigerweise von der Art der Fermentation ab und wird im Einzelfall zweckmässigerweise in einer dem Fachmann bekannten Weise festgelegt. Beispielsweise eignen sich zur Durchführung des erfindungsgemässen Verfahrens Glukose- oder Saccharosekonzentrationen im Bereich von ca. 5 bis ca. 50 g/L, bevorzugt sind Konzentrationen im Bereich von ca. 10 bis ca. 20 g/L. Das als Stickstoffquelle bevorzugte Ammoniumsulfat oder Ammoniumnitrat setzt man im Bereich von ca. 0.03 bis ca. 5 g/L ein, besonders von ca. 0.06 bis ca. 2 g/L.

Die verwendeten Mineralsalze werden zweckmässigerweise im Bereich von ca. 0.05 bis ca. 10 g/L, insbesondere von ca. 0.1 bis ca. 5 g/L eingesetzt. Die Spurenelemente verwendet man in der Regel in einem Konzentrationsbereich von jeweils ca. 0.005 bis ca. 20 mg/L, besonders von ca. 0.01 bis ca. 5 mg/L, z.B. eingesetzt als wässrige Lösung.

Die als Aminosäurenquelle verwendeten Proteinhydrolysate werden zweckmässigerweise im Bereich von ca. 5 bis ca. 50 g/L, insbesondere von ca. 10 bis ca. 20 g/L eingesetzt. Den Zusatz von freien Aminosäuren, wie Leucin, Methionin oder Phenylalanin wählt man zweckmässigerweise im Bereich von ca. 0.1 bis ca. 5 g/L, insbesondere von ca. 0.2 bis ca. 2 g/L.

Das für die Züchtung von *Neurospora crassa* notwendige Vitamin Biotin setzt man zweckmässigerweise im Bereich von ca. 1 bis ca. 20 µg/L ein, besonders von ca. 2 bis ca. 5 µg/L.

Die Fermentationstemperatur hängt in wesentlichen vom verwendeten Mikroorgansimus ab und bewegt sich

zweckmässigerweise im Bereich von ca. 10 bis ca. 50 °C, insbesondere von ca. 20 bis ca. 37 °C. Der pH-Wert des Nährmediums liegt normalerweise im Bereich von ca. 4 bis ca. 10, vorzugsweise von ca. 5 bis ca. 8. Die bevorzugte Fermentationsdauer-nämlich bis zur Beendigung des Wachstums - liegt zwischen ca. 10 und ca. 100 Stunden, insbesondere zwischen ca. 18 und ca. 72 Stunden.

Nach der Fermentation werden die Zellen zweckmässigerweise mechanisch vom Nährmedium getrennt, gewaschen und direkt für die Transformation eingesetzt. Für die Umsetzung von L-4-Hydroxyisoleucin (II) können aber auch immobilisierte Zellen oder aus Zellen hergestellte Rohextrakte, sowie - z.B. isolierte - Präparate von L-Aminosäureoxidase in freier oder immobilisierter Form verwendet werden. Da der Umsetzung ein oxidativer Prozess zugrunde liegt, wird das Reaktionsgemisch vorzugweise intensiv geschüttelt oder zusätzlich belüftet.

Die Isolierung des Enzyms aus den Zellen, falls erwünscht, kann in dem Fachmann bekannter Weise erfolgen, also z.B. mittels mechanischem Aufschluss, und Reinigung, z.B. Ionenaustauschchromatographie und/oder Adsorptionschromategraphie, etc.

Die Biotransformation wird vorzugsweise in einem pH-Bereich von ca. 4 bis ca. 8, insbesondere von ca. 5 bis ca. 7, durchgeführt. Ueblicherweise erfolgt die erfindungsgemässe Umsetzung bei einer Temperatur von ca. 10 bis ca. 40°C, vorzugsweise bei 20 bis 37°C. Das Edukt L-4-Hydroxyisoleucin (II) wird in einer Menge von ca. 0.5 bis ca. 5 % (w/v), bevorzugt von ca. 1 bis ca. 2 %, in der Reaktionslösung eingesetzt.

Das Verhältnis von (II): Enzymmenge wird zweckmässigerweise so gewählt, dass die Reaktion nicht zu viel Zeit beansprucht, also z.B. ca. 6 - ca. 24 Stunden nicht überschreitet, was mit wenigen Versuchen abgeklärt werden kann.

Nach abgeschlossener Umsetzung, deren Verlauf durch (konventionelle) Aminosäurenanalyse (auf die erfolge Konsumption von II)verfolgt werden kann, wird das Reaktionsgemisch in für $\gamma$-Hydroxysäuren bekannter Weise lactonisiert, also zweckmässigerweise mit Säure, z.B. mit verdünnter Phosphorsäure, auf einen pH-Wert von ca. 1 bis ca. 3, bevorzugt von ca. 1.5 bis ca. 2.5, angesäuert und anschliessend durch Erhitzen lactonisiert. Die Hitzebehandlung erfolgt zweckmässigerweise in einem Temperaturbereich zwischen ca. 70 bis ca. 95°C, vorzugsweise bei ca. 80°C während ca. 20 bis ca. 90 Minuten, bevorzugt ca. 30 bis ca. 60 Minuten.

Die Lösung kann mit einem organischen Lösungsmittel wie einem Ester, besonders Essigsäureeethylester oder einem Ether wie Methyl-tert.butylether nach bekannten Methoden, beispielsweise in einer Gegenstromextraktionsanlage, extrahiert werden. Anschliessend wird das Lösungsmittel zweckmässigerweise destillativ entfernt und das Produkt in einem in der Aromenindustrie geläufigen Träger aufgenommen, wie dies z.B. Oele, vor allem Erdnüssoel oder z.B. Triethylcitrat, sind.

Das nach der biotechnologischen Umsetzung von (II) anfallende zellfreie Reaktionsgemisch mit einem Gehalt an (I) kann aber auch direkt mit einem Träger wie Maltodextrin oder einem anderen Stärkederivat gemischt und (sprüh) getrocknet werten. Dieses Produkt kann als Aromenvorläufer von (III) für die Aromatisierung ausgesuchter Lebensmittelprodukte eingesetzt werden. Es handelt sich dabei um Lebensmittelprodukte, die einer thermischen Behandlung unterworfen werden. Eine Hitzebehandlung (Kochen, Backen, Fritieren, Grillieren, Rösten, Behandlung mit Mikrowellen usw.) erfolgt zweckmässigerweise in einem Temperaturbereich zwischen ca. 80 und ca. 250°C während ca. 2 bis ca. 30 Minuten. Die Dosierung des Vorläufers (I) kann beispielsweise zwischen ca. 1 ppb bis ca. 5 ppm (im Endprodukt) variiert werden.

Die folgenden Beispiele erläutern die vorliegende Erfindung.

<u>Beispiel 1</u>

<u>Isolierung und Charakterisierung von 2-Keto-3-methyl-4-hydroxy-valeriansäure (= Hydroxy-3-methyl-2-keto-pentansäure) (I)</u>

a) Isolierung von L-4-Hydroxyisoleucin (II) aus Samen von Bockshornklee *Trigonella foenum graecum L.*

Die Extraktion und Reinigung von L-4-Hydroxyisoleucin wurde nach L. Fowden, H.M. Pratt und A. Smith (*Phytochemistry* (1973), **12**, 1707) durchgeführt. Aus 4 kg Samen von *Trigonella foenum graecum L.* wurden 12 g gereinigtes L-4-Hydroxyisoleucin isoliert (Ausbeute 62 %). Reinheit: > 97 % (Aminosäuren-analyse). Optische Drehung: $[\alpha]_D^{20}$ = + 32.3 °(c 1 (d.h. 1 g/100 ml), $H_2O$); Literaturwert: 31.0°.

b) Biotransformation von L-4-Hydroxyisoleucin (II) mit *Morganella morganii*

*Morganella morganii* (DSM No. 30117) wurde im Nährmedium A (Tabelle 1) in einem Fermenter bei pH 7 und einer Temperatur von 30°C während 20 Stunden kultiviert. Anschliessend wurden die Zellen zentrifugiert, mit 0.02 M Natriumphosphatpuffer (pH 7.0) gewaschen und in 0.05 M Natriumphosphatpuffer pH 7.0 resuspendiert und es wurde auf eine optische Dichte von 20 bei 650 nm (bzw. O.D. 0,2 nach 100-facher Verdünnung) eingestellt. Diese Zellsuspension wurde in einen Schüttelkolben gegeben und mit 7.7 mmol L-4-Hydroxyisoleucin pro Liter versetzt. Nach einer Inkubation von 6 Stunden bei 30°C auf einer Schüttelmaschine war die vorgelegte Menge L-4-Hydroxyisoleucin vollständig umgesetzt. Die Zellen wurden durch Zentrifugation vom Reaktionsgemisch abgetrennt und

der erhaltene Ueberstand lyophilisiert.

Tabelle 1

| Nährmedium A | |
|---|---|
| Glucose | 10.0 g/L |
| Caseinhydrolysat | 10.0 g/L |
| $Na_2HPO_4 \cdot 2H_2O$ | 11.9 g/L |
| $KH_2PO_4$ | 4.5 g/L |
| $(NH_4)_2SO_4$ | 1.0 g/L |
| $MgSO_4 \cdot 7H_2O$ | 0.1 g/L |
| $CaCl_2 \cdot 2H_2O$ | 4 mg/L |
| $FeSO_4 \cdot 7H_2O$ | 0.5 mg/L |

c) Isolierung des Natriumsalzes von 2-Keto-3-methyl-4-hydroxyvaleriansäure (I)

Der gefriergetrocknete Rückstand wurde mit Methanol extrahiert und das Natriumsalz von (I) in üblicher Weise durch Zugabe von Methyl-tert.butylether ausgefällt. Die bisher unbekannte Substanz zeigte die folgenden physikalischen Eigenschaften:

| | |
|---|---|
| Schmelzpunkt: | 144°C (Zers.) |
| Optische Drehung: | $[\alpha]_D^{20}$ : +18.49° (c 0.84 in Methanol) |
| IR: | 1720 $cm^{-1}$, 1630 $cm^{-1}$, 1390 $cm^{-1}$, 1130 $cm^{-1}$ |
| $^1$H-NMR: | $\delta$=4.18 ppm (2 x q), 3.18 (2 x q), 1.20 (2 x d), 1.05 (2 x d) |
| MS: | (Elektronenspray-Ionisation) 145.1 ($M^-$), 101.1, 57.2 313.1 ($2M^-Na^+$), 481.3 ($3M^-2Na^+$), 649.4 ($4M^-3Na^+$), 817.6 ($5M^-4Na^+$), (Anionen-Clusters). |

d) Umsetzung von 2-Keto-3-methyl-4-hydroxy-valeriansäure (I) in (5S)-3-Hydroxy-4,5-dimethyl-2(5H)-furanon (III)

840 mg des Natriumsalzes von (I) wurden in 50 mL $H_2O$ gelöst und mit verdünnter Phosphorsäure auf pH 2.0 gebracht. Die Lösung wurde während 30 Minuten auf 85°C gehalten und nach Erkalten mit 2 x 50 ml Essigsäureethylester extrahiert. Nach Entfernen des Lösungsmittels fielen 320 mg (Ausbeute 50%) von (III) an. Die mit präparativer Dünnschichtchromatographie (Kieselgel 60) gereinigte Substanz (III) zeigte eine optische Drehung von: $[\alpha]_D^{20}$ : 21.66° (c 1.0 in Chloroform).

Beispiel 2

Zellen von *Morganella morganii* wurden, wie in Beispiel 1 beschrieben, in einem Fermenter gezüchtet, zentrifugiert, gewaschen und anschliessend auf eine Dichte von 20 (650 nm) eingestellt. Nach Zugabe von 7.7 mmol L-4-Hydroxyisoleucin pro Liter wurde die Zellsuspension in einem Erlenmeyerkolben bei 30°C geschüttelt. Nach 18 Stunden war die vorgelegte Menge L-4-Hydroxyisoleucin vollständig umgesetzt. Die Zellen wurden durch Zentrifugation vom Reaktionsgemisch abgetrennt und der klare Ueberstand mit verdünnter Phosphorsäure auf pH 2.5 eingestellt. Nach Erhitzen (30 Minuten, 80°C) wurde die Lösung 2 mal mit dem halben Volumen von Essigsäureethylester extrahiert. Nach Entfernung des Lösungsmittel fiel ein gelbliches Oel an. Ausbeute von (III): 65%.

Beispiel 3

Gemäss Beispiel 1 wurden Zellen von *Morganella morganii* in einem Fermenter gezüchtet, zentrifugiert und anschliessend mit Wasser gewaschen. Die Zellen wurden in 2% Natriumalginat resuspendiert und bei Raumtemperatur tropfenweise in eine gerührte Lösung von $CaCl_2$ (2%) eingetragen. Die in kleinen Kugeln anfallenden immobilisierten Zellen wurden gründlich mit Wasser gewaschen und anschliessend für die Umsetzung von L-4-Hydroxyisoleucin verwendet. Dabei wurde ein Teil sedimentierte, immobilisierte Zellen mit 2 Teilen einer Lösung von L-4-Hydroxyisoleucin versetzt (11.25 mmol/L) und bei 30°C in einem Erlenmeyerkolben geschüttelt. Nach 24 Stunden war war die vorgelegte Menge 4-Hydroxyisoleucin vollständig umgesetzt. Die immobilisierten Zellen wurden über eine Nutsche vom Reaktionsgemisch abgetrennt und mit Wasser sorgfältig gewaschen. Das klare Filtrat wurde wie in Beispiel 2 weiter behandelt. Ausbeute an (III): 60%.

Beispiel 4

*Providencia rettgeri* (ATCC 9250) wurde im Nährmedium A (Tabelle 1) in einem Fermenter bei pH 7 und einer Temperatur von 30°C während 20 Stunden gezüchtet. Anschliessend wurden die Zellen zentrifugiert, mit 0.02 M Natriumphosphatpuffer pH 7.0 gewaschen und in 0.1 M Natriumphosphatpuffer pH 7.2 resuspendiert und eine optische Dichte von 20 bei 650 nm eingestellt. Diese Zellsuspension wurde in einen Schüttelkolben gegeben und mit 7.7 mmol 4-Hydroxyisoleucin pro Liter versetzt. Nach einer Inkubation bei 30°C von 18 Stunden auf einer Schüttelmaschine war die zugegebene Menge L-4-Hydroxyisoleucin vollständig umgesetzt. Die Zellen wurden durch Zentrifugation vom Reaktionsgemisch abgetrennt und der Ueberstand wie in Beispiel 2 weiter behandelt. Ausbeute an (III): 60%.

Beispiel 5

*Neurospora crassa* (FGSC 988) wurde im Nährmedium B (Tabelle 2) in 2 Liter Schüttelkolben bei pH 6.0 und einer Temperatur von 25°C während 48 Stunden gezüchtet. Das Mycel wurde durch 4 Lagen Baumwollgaze filtriert, mit Wasser gut gewaschen und gefriergetrocknet und anschliessend mit einem Homogenisator fein pulversiert. 5 g Mycelpulver wurden mit 50 mL 0.1 M Natriumphosphatpuffer pH 7.2 vermischt und bei 4°C während 30 Minuten gerührt. Die unlöslichen Zellbestandteile wurden durch Zentrifugation entfernt und der klare Ueberstand mit 7.7 mmol L-4-Hydroxyisoleucin pro Liter versetzt. Nach einer Inkubationzeit von 18 Stunden bei 30°C war die vorgelegte Menge L-4-Hydroxyisoleucin vollständig umgesetzt. Die Umwandlung der gebildeten 4-Hydroxyketosäure (I) in (III) erfolgte gemäss Beispiel 2. Ausbeute: 60%.

Tabelle 2

| Nährmedium B | |
|---|---|
| Saccharose | 20.0 g/L |
| Natriumcitrat · 5 $H_2O$ | 3.0 g/L |
| $KH_2PO_4$ | 5.0 g/L |
| $(NH_4)NO_3$ | 0.08 g/L |
| $MgSO_4 \cdot 7H_2O$ | 0.2 g/L |
| $CaCl_2 \cdot 2H_2O$ | 0.1 g/L |
| L-Phenylalanin | 0.73 g/L |
| $ZnSO_4 \cdot 7\ H_2O$ | 5.0 mg/L |
| $Fe(NH_4)_2(SO_4)_2 \cdot 6H_2O$ | 1.0 mg/L |
| $CuSO_4 \cdot 5\ H_2O$ | 0.25 mg/L |
| $MnSO_4 \cdot 1\ H_2O$ | 0.05 mg/L |
| $H_3BO_3$ | 0.05 mg/L |
| $Na_2MoO_4 \cdot 2\ H_2O$ | 0.05 mg/L |

Beispiel 6

Der in Beispiel 5 beschriebene Rohextrakt wurde zur kovalenten Immobusierung von L-Aminosäureoxidase verwendet. 50 ml Extrakt wurde gegen 1.0 M Kaliumphosphatpuffer pH 7.5 dialysiert und mit 10 g Acrylharzperlen Eupergit® (Röhm Pharma, Weiterstadt, Deutschland) vermischt. Diese Suspension wurde während 24 Stunden bei Raumtemperatur stehengelassen und das Harz anschliessend mit 2 L 0.1 M Kaliumphosphatpuffer pH 7.2 gewaschen. Das immobilisierte Enzympräparat wurde, wie in Beispiel 3 beschrieben, mit L-4-Hydroxyisoleucin (7.7 mM) während 24 Stunden bei 30°C umgesetzt. Die Trennung des immobilisierten Enzyms vom Reaktionsgemisch erfolgte durch Abnutschen, und der Ueberstand wurde wie in Beispiel 2 beschrieben weiter behandelt. Die Ausbeute an (III) betrug 55%.

Beispiel 7

Ahornsiruparoma

Zwei Ahornsiruparomen wurden gemäss folgender Zusammensetzung hergestellt:

| | Gewichtsteile | |
|---|---|---|
| | A | B |
| Peru Balsam | 5 | 5 |
| Vanilla Extrakt | 50 | 50 |
| Buttersäure (natürlich) | 5 | 5 |
| Furonol (natürlich 15%) | 10 | 10 |
| Röstbase (roast base) natürlich | 5 | 5 |
| III (0,1% in Triacetin) gemäss Beispiel 2 | - | 150 |
| Triacetin | 925 | 775 |
| | 1000 | 1000 |

Ein Vergleich der obigen Aromen zeigt, dass die Anwesenheit von 150 Teilen von (III) in B diesem Aroma den typischen süssen, karamellartigen würzigen Charakter von Ahornsirup verleiht und zur Verstärkung des Körpers wesentlich beiträgt. Dieses Aroma kann z.B. in einem Milchdrink in einer Konzentration von 0,1% eingesetzt werden.

Beispiel 8

| Walnussaroma | | |
|---|---|---|
| | Gewichtsteile | |
| | A | B |
| Kakaoschalenextrakt | 50 | 50 |
| Vanillaextrakt | 10 | 10 |
| Oelsäure | 30 | 30 |
| Walnussbase (natürlich) | 10 | 10 |
| III (0,1% in Triacetin) gemäss Beispiel 2 | | 120 |
| Propylenglykol | 900 | 780 |
| | 1000 | 1000 |

Ein Vergleich der obigen Aromen zeigt, dass die Anwesenheit von 120 Teilen (III) in B diesem Aroma den typischen Walnussaromacharakter verleiht und zur Verstärkung der Nuss- und Fettnoten wesentlich beiträgt. Dieses Aroma kann z.B. in einem Milchdrink in einer Konzentration von 0,1% eingesetzt werden.

Beispiel 9

HVP-freie Braune Sauce

Eine HVP-freie braune Sauce wurde gemäss folgender Zusammensetzung hergestellt:

| Bestandteil | Gewichtsteile |
|---|---|
| Weissmehl | 370 |
| Rinderfett | 145 |
| Modifizierte Stärke | 145 |
| Hefeextrakt | 97 |
| Kochsalz | 80 |
| Tomatenpulver | 60 |
| Maltodextrin | 53,7 |
| Mononatriumglutamat | 30 |
| Karamelpulver | 10 |
| Kurkuma | 4,5 |
| Gewürzmischung (Spice'N'Easy®) (Zwiebel-, Knoblauch- und Rosmarinextrakt) | 4,8 |

(fortgesetzt)

| Bestandteil | Gewichtsteile |
|---|---|
| | 1000 |

Beim Erhitzen der Sauce in Anwesenheit des Aromenvorläufers (I) (2 ppm) wird der Geschmack deutlich in Richtung Fleisch, Bouillon, HVP und Salz verstärkt.

Beispiel 10

| Crackers | |
|---|---|
| Bestandteil | Gewichtsteile |
| Weissmehl | 575 |
| Pflanzenfett | 150 |
| Magermilchpulver | 37 |
| Puderzucker | 15 |
| Süssmolkepulver | 10,2 |
| Hefeautolysat | 10,0 |
| Ammoniumbicarbonat | 10,0 |
| Kochsalz | 10,0 |
| Natriumhydrogenpyrophosphat | 1,0 |
| Weisser Pfeffer | 0,4 |
| Paprika | 0,4 |
| Wasser | 181 |
| Aromenvorläufer (I) | 1 ppm |

Beim Backen des Crackerteigs (230°C, 10 Min.) in Anwesenheit des Aromenvorläufers (I) wird das Raumaroma (room flavour) und der Geschmack des Produkts in Richtung würzig, bouillonartig und krautig verstärkt.

**Patentansprüche**

1. 4S-Hydroxy-3-methyl-2-ketopentansäuren der Formel (I)

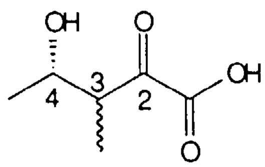

(I)

2. Verfahren zur Herstellung der Verbindungen gemäss Anspruch 1, dadurch gekennzeichnet, dass man L-4-Hydroxyisoleucin (2S,3R,4S) mit einer L-Aminosäureozidase bzw. einem dieses Enzym enthaltenden Mikroorganismus umsetzt.

3. Verfahren zur Herstellung von optisch aktivem (5S)-3-Hydroxy-4,5-dimethyl-2(5H)-furanon der Formel (III)

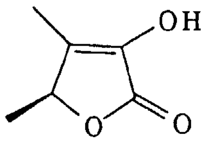

(III)

dadurch gekennzeichnet, dass man eine Verbindung der Formel (I)

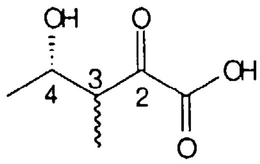

(I)

in an sich bekannter Weise lactonisiert.

4. Verwendung der Verbindungen (I) gemäss Anspruch 1 als Aromenvorläufer.

## Claims

1. 4S-Hydroxy-3-methyl-2-ketopentanoic acids of formula (I)

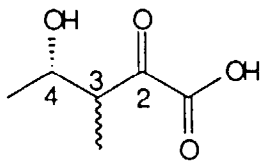

(I)

2. A process for the manufacture of the compounds according to claim 1, characterized by reacting L-4-hydroxy-isoleucine (2S,3R,4S) with a L-amino acid oxidase or a microorganism containing this enzyme.

3. A process for the production of optically active (5S)-3-hydroxy-4,5-dimethyl-2(5H)-furanone of formula (III)

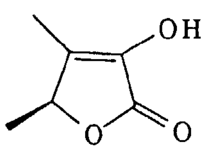

(III)

characterized by lactonizing a compound of formula (I)

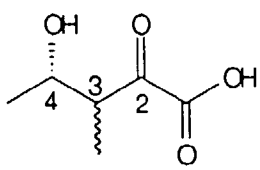

(I)

in a manner known per se.

**4.** The use of compounds (I) according to claim 1 as flavor precursors.

**Revendications**

**1.** Acides 4S-hydroxy-3-méthyl-2-cétopentanoïques de formule I

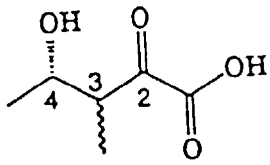

(I)

**2.** Procédé de préparation des composés selon la revendication 1, caractériséen ce que l'on fait réagir la L-4-hydroxyisoleucine (2S, 3R, 4S) avec une L-aminoacide-oxydase ou avec un microorganisme contenant cette enzyme.

**3.** Procédé de préparation de la (5S)-3-hydroxy-4,5-diméthyl-2(5H)-furannone possédant l'activité optique et répondant à la formule III

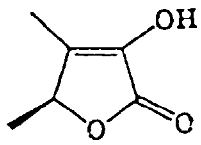

(III)

caractérisé en ce que l'on lactonise de manière connue en soi un composé de formule I

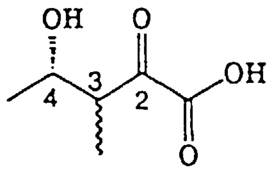

(I)

**4.** Utilisation des composés I selon revendication 1 en tant que précurseurs pour des substances gustatives.